# EUROPEAN PATENT APPLICATION

(11) **EP 4 652 996 A1**
(43) Date of publication of application: **26.11.2025**
(21) Application number: 24744425.0
(22) Date of filing: 05.02.2024
(51) Int. Cl.: A61K 31/517, C07D 405/14, A61K 31/704, C12N 15/12, A61P 35/00, A61P 25/00

(54) **USE OF QUINAZOLINE COMPOUND IN OVERCOMING OSIMERTINIB DRUG RESISTANCE**

(30) Priority: 16.01.2023 CN 202310079445
(71) Applicant: WEISHANG (SHANGHAI) BIO-PHARMACEUTICAL CO., LTD., Shanghai 201100 (CN)
(72) Inventor: ZHONG, Wei, Shanghai 201100 (CN)
(74) Representative: Huang, Liwei
(86) International application number: PCT/CN2024/076045
(87) International publication number: WO 2024/153256

(57) **Abstract**

A use of at least one of a quinazoline derivative as represented by formula I, and a salt, prodrug, prodrug salt, solvate, hydrate and polymorph thereof in overcoming Osimertinib drug resistance, relating to the technical field of medicine and health. Cell experiments and kinase inhibition tests improve that the described compounds overcome Osimertinib drug resistance caused by double mutation of EGFR C797S, L792H, and Del19/C797S or double mutation of L858R/C797S, thereby providing a means for medical and healthy use for treating non-small cell lung cancer and neural center transfer thereof.

## Description

### Technical Field

The present invention relates to the use of a quinazoline compound in overcoming osimertinib resistance, belonging to the field of biopharmaceutical technology.

### Background Art

The quinazoline derivative with the molecular formula C₂₃H₂₁F₃N₄O₂, chemical name (R)-6-[(3,3-difluoro-1-methylpiperidin-4-yl)oxy]-N-(3-ethynyl-2-fluorophenyl)-7-methoxyquinazolin-4-amine (I), is a targeted anticancer drug capable of crossing the blood-brain barrier. It is a highly selective epidermal growth factor receptor (EGFR) tyrosine kinase inhibitor and can be used to treat brain or leptomeningeal metastases of NSCLC, head and neck squamous cell carcinoma, squamous cell carcinoma, brainstem tumors, primary brain cancer, gliomas, or other cancers. The Chinese patent 201610982608.6 describes a preparation method and application of the said quinazoline derivative (I) (molecular formula C₂₃H₂₁F₃N₄O₂). As known to those skilled in the art, resistance mutations arise after treatment with osimertinib (also known as Osimertinib, AZD9291 or Tagrisso), preventing effective tumor growth inhibition by osimertinib. Therefore, developing inhibitors that can overcome these resistance mutations is highly valuable and carries significant implications.

### Summary of the Invention

The objective of the present invention is to provide the use of a quinazoline compound in overcoming osimertinib resistance.

The objective of the present invention is achieved through the following technical solutions:
The present invention provides the use of a quinazoline compound in the preparation of a medicament for overcoming osimertinib resistance.

As a preferred embodiment, the said quinazoline compound includes the quinazoline derivative of formula I, at least one of its salts, prodrugs, and prodrug salts, and at least one solvate, hydrate, and polymorph of the said salts.

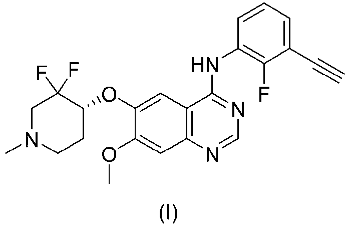

As a preferred embodiment, the said quinazoline compound includes hydrochloride, sulfate, maleate, succinate, adipate, glycolate, malate, fumarate, benzenesulfonate, benzoate, hippurate, and oxalate of the quinazoline derivative, as well as their solvates, hydrates, and polymorphs.

As a preferred embodiment, the medicament is used for the treatment of osimertinib-resistant NSCLC (non-small cell lung cancer) and NSCLC with CNS (central nervous system) metastases.

As a preferred embodiment, NSCLC with CNS metastases includes brain metastases or leptomeningeal metastases of NSCLC.

As a preferred embodiment, the said osimertinib resistance is driven by the EGFR C797S mutation, L792H mutation, Del19/C797S dual mutations, or L858R/C797S dual mutations.

Compared with the prior art, the present invention has the following advantageous effects:
1. The quinazoline derivative (I) and pharmaceutically acceptable salts thereof described in the present invention can inhibit tumor growth by overcoming osimertinib resistance especially driven by the EGFR C797S mutation, L792H mutation, Del19/C797S dual mutations, or L858R/C797S dual mutations.
2. The quinazoline derivative (I) and pharmaceutically acceptable salts thereof described in the present invention can effectively treat osimertinib-resistant NSCLC and NSCLC with CNS metastases, including brain metastasis or leptomeningeal metastasis, driven by the aforementioned resistance mutations.

### Brief Description of Drawings

The features, objectives, and advantages of the present invention will become more apparent through detailed description of the non-limiting embodiments, with reference to the accompanying drawings:

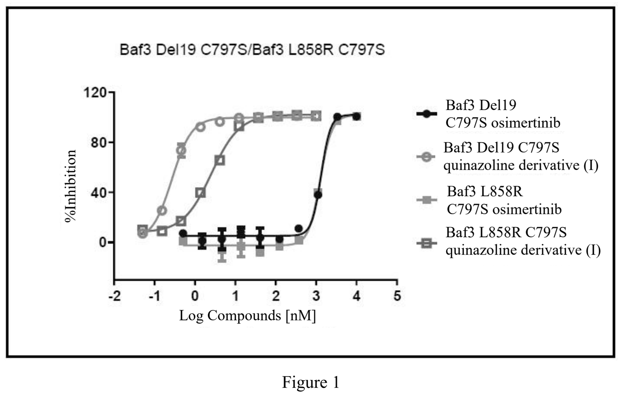

Figure 1 shows the antiproliferative effect in Ba/F3 L858R/C797S and Del19/C797S cells compared to osimertinib.

### Detailed Description

The present invention will be described in detail below with reference to specific embodiments. The following embodiments will help those skilled in the art better understand the present invention, but do not in any way limit the scope of the invention. It should be noted that, for those skilled in the art, several modifications and improvements can be made without departing from the spirit of the present invention. These are all within the scope of protection of the present invention.

### Example 1

Inhibition of Ba/F3 L858R/C797S and Del19/C797S cell growth by quinazoline derivative (I) described in the present invention compared to osimertinib in the antiproliferative activity assay
The test compounds in DMSO were added into a 384-well plate at 0.05, 0.15, 0.45, 1.37, 4.11, 12.3, 37.0, 111.1, 333.3, and 1000 nM, followed by the addition of cell suspension into the same plate at 700 cells/30 µL/well. After incubating the cells in a 5% CO₂ incubator at 37°C for 72 hours, 25 µL of CellTiter-Glo reagent (CTG) was added to each well and the plate was gently shaken and incubated in a 5% CO₂ incubator at 37°C (protected from light) for 30 minutes. Fluorescence was measured using the Envision reader. Antiproliferative activity was evaluated using IC₅₀ (half-maximal inhibitory concentration) and relative potency (%): Relative potency % = Osimertinib IC₅₀ / Quinazoline derivative (I) IC₅₀ × 100%.

As shown in Figure 1, in the antiproliferative activity assay using Ba/F3 L858R/C797S and Del19/C797S cells, the quinazoline derivative (I) described in the present invention overcame osimertinib resistance and exhibited strong inhibition of cell proliferation driven by the osimertinib-resistant EGFR L858R/C797S and Del19/C797S mutations compared to osimertinib. The relative bioactivity thereof was approximately 490 times and 4900 times that of osimertinib, respectively.

| Cell line | Osimertinib IC₅₀ (nM) | Quinazoline derivative (I) IC₅₀ (nM) | Relative potency % |
|---|---|---|---|
| Ba/F3 L858R/C797S | 1255.0 | 2.561 | 49004.3 |
| Ba/F3 Del19/C797S | 1324.0 | 0.27 | 490370.4 |

### Example 2

### Inhibition of EGFR C797S, L792H, L858R/C797S, and Del19/C797S kinases by quinazoline derivative (I) described in the present invention compared to osimertinib

The test compounds in DMSO were added into a 384-well plate at 0.017, 0.05, 0.15, 0.45, 1.37, 4.11, 12.3, 37.0, 111.1, and 333.3 nM. At room temperature, 5 µL each of kinase, peptide substrate (TK-substrate-biotin), and ATP were added to the plate and incubated at room temperature for 40 minutes. Then, 5 µL each of Sa-XL 665 HTRF detection buffer and TK-antibody-Cryptate were added to the plate and incubated at room temperature for 1 hour. Fluorescence at 615 nm (Cryptate) and 665 nm (XL665) was measured using the Envision 2104 microplate reader. Kinase inhibition was evaluated using IC50 (half-maximal inhibitory concentration) and relative potency (%): Relative potency % = Osimertinib IC₅₀ / Quinazoline derivative (I) IC₅₀ × 100%.

In the kinase inhibition assay using EGFR C797S, L792H, L858R/C797S, and Del19/C797S kinases, the quinazoline derivative (I) described in the present invention overcame osimertinib resistance and exhibited strong inhibition of the osimertinib-resistant EGFR C797S, L792H, L858R/C797S, and Del19/C797S kinases compared to osimertinib. The relative bioactivity thereof was approximately >1052 times, 49 times, 93118 times, and 498 times that of osimertinib, respectively.

| Kinase mutation | Osimertinib IC₅₀ (nM) | Quinazoline derivative (I) IC₅₀ (nM) | Relative potency % |
|---|---|---|---|
| EGFR C797S | >1000 | 0.95 | >105263 |
| EGFR L792H | 168.4 | 3.424 | 4918 |
| EGFR L858R/C797S dual mutations | 3423 | 0.037 | 9311751.9 |
| EGFR Del19/C797S dual mutations | 65.2 | 0.131 | 49809.0 |

The quinazoline derivative (I) described in the present invention overcomes osimertinib resistance. Compared to osimertinib, the said derivative shows strong inhibition of osimertinib-resistant EGFR C797S, L792H, L858R/C797S, and Del19/C797S mutations. It can overcome resistance caused by the EGFR C797S mutation, L792H mutation, Del19/C797S dual mutations or L858R/C797S dual mutations, thereby inhibiting tumor growth. It effectively treats osimertinib-resistant NSCLC, including NSCLC with CNS metastasis, driven by the said resistance mutations.

The above describes the specific embodiments of the present invention. It should be understood that the present invention is not limited to the above specific embodiments, and those skilled in the art can make various modifications or changes within the scope of the claims, without departing from the essence of the invention.

## Claims

1. Use of a quinazoline compound in preparing a medicament for overcoming osimertinib resistance.

2. The use according to claim 1, wherein the quinazoline compound includes at least one selected from a quinazoline derivative of formula I, a salt thereof, a prodrug thereof, a prodrug salt thereof, a solvate thereof, a hydrate thereof, and a polymorph thereof.

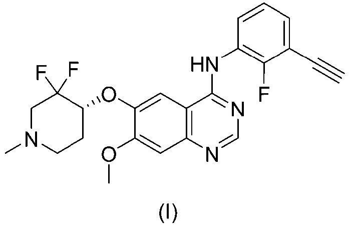

3. The use according to claim 2, wherein a salt of the quinazoline compound include hydrochloride, sulfate, maleate, succinate, adipate, glycolate, malate, fumarate, benzenesulfonate, benzoate, hippurate, or oxalate of the quinazoline derivative; the quinazoline compound includes a solvate, a hydrate, or a polymorph of the salt.

4. The use according to claim 1, wherein the medicament is used for treating osimertinib-resistant non-small cell lung cancer and central nervous system metastasis of non-small cell lung cancer.

5. The use according to claim 4, wherein central nervous system metastasis of non-small cell lung cancer includes brain metastasis or leptomeningeal metastasis of non-small cell lung cancer.

6. The use according to claim 1, wherein the osimertinib resistance is caused by EGFR C797S mutation, L792H mutation, Del19/C797S dual mutations, or L858R/C797S dual mutations.
